**Europäisches Patentamt**

**European Patent Office**

**Office européen des brevets**

(19)

(11) Veröffentlichungsnummer : **0 349 861 B1**

(12)

# EUROPÄISCHE PATENTSCHRIFT

(45) Veröffentlichungstag der Patentschrift :
**26.08.92 Patentblatt 92/35**

(51) Int. Cl.⁵ : **C07D 313/04**

(21) Anmeldenummer : **89111531.3**

(22) Anmeldetag : **24.06.89**

(54) **Verfahren zur Herstellung von Caprolacton.**

(30) Priorität : **08.07.88 DE 3823213**

(43) Veröffentlichungstag der Anmeldung :
**10.01.90 Patentblatt 90/02**

(45) Bekanntmachung des Hinweises auf die
Patenterteilung :
**26.08.92 Patentblatt 92/35**

(84) Benannte Vertragsstaaten :
**BE CH DE ES FR GB IT LI NL**

(56) Entgegenhaltungen :
**BE-A- 693 971**
**GB-A- 1 115 702**

(56) Entgegenhaltungen :
**CHEMICAL ABSTRACTS, Band 78, Nr. 20, 21.**
**Mai 1973, Seite 18, Zusammenfassung Nr.**
**125138t, Columbus, Ohio, US; & JP-A-7229 505**
**(ASAHI CHEMICAL INDUSTRY CO., LTD) 02-**
**08-1972**

(73) Patentinhaber : **BASF Aktiengesellschaft**
**Carl-Bosch-Strasse 38**
**W-6700 Ludwigshafen (DE)**

(72) Erfinder : **Richter, Wolfgang, Dr.**
**Am Hüttenwingert 16**
**W-6706 Wachenheim (DE)**
**Erfinder : Fischer, Rolf, Dr.**
**Bergstrasse 98**
**W-6900 Heidelberg (DE)**
**Erfinder : Vagt, Uwe, Dr.**
**Im Vogelgesang 89**
**W-6720 Speyer (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der US-PS 3 189 619 ist ein Verfahren bekannt, bei dem man 6-Hydroxycapronsäureester in einer ersten Stufe mit Trialkylborat umsetzt und dann das Umsetzungsprodukt durch Erhitzen auf 200 bis 250°C unter vermindertem Druck in Caprolacton überführt. Dieses Verfahren ist für die technische Ausführung aufwendig und ungeeignet.

Nach einem anderen, aus der FR-PS 1 474 903 bekannten Verfahren werden 6-Hydroxycapronsäureester in Gegenwart von Oxiden wie Magnesiumoxid, Zinkoxid, Cadmiumoxid, Aluminiumoxid oder Titandioxid in flüssiger Phase unter vermindertem Druck auf eine Temperatur von 150 bis 350°C erhitzt und Caprolacton fortlaufend abdestilliert. Dieses Verfahren hat den Nachteil, daß die verwendeten Katalysatoren rasch inaktiv werden und nur unter größerem Aufwand regeneriert werden können.

Aus der GB-A-1,115,702 ist bekannt, daß man ω-Acyloxyfettsäuren in der Gasphase über basische Katalysatoren leitet und entsprechende Lactone erhält. Darüber hinaus wird in Chemical Abstracts 78, 125138 t (1973) beschrieben, daß man Ethylhydroxycaproat über Zn als Katalysator leitet und das entsprechende Lacton erhält.

Es war deshalb die technische Aufgabe gestellt, ein Verfahren zur Herstellung von Caprolacton aus 6-Hydroxycapronsäureestern zur Verfügung zu stellen, das einfach kontinuierlich durchführbar ist und bei dem die verwendeten Katalysatoren eine längere Lebensdauer haben und leicht regenerierbar sind.

Diese Aufgabe wird gelöst in einem Verfahren zur Herstellung von ε-Caprolacton, bei dem man 6-Hydroxycapronsäureester in Gegenwart von oxidischen Katalysatoren auf eine Temperatur von 150 bis 450°C erhitzt, dadurch gekennzeichnet daß man dampfförmigen 6-Hydroxycapronsäureester zusammen mit einem inerten Trägergas über fest angeordnetes oder in auf- und abwirbelnder Bewegung befindliches Siliciumdioxid als Katalysator leitet.

Das neue Verfahren hat den Vorteil, daß es auf einfache Weise kontinuierlich durchgeführt werden kann. Weiter hat das neue Verfahren den Vorteil, daß der verwendete Katalysator nicht mit Rückständen belastet wird, eine längere Lebensdauer hat und auf einfache Weise regenerierbar ist.

Bevorzugte 6-Hydroxycapronsäureester leiten sich von Alkanolen mit 1 bis 12 Kohlenstoffatomen, Cycloalkanolen mit 5 bis 7 Kohlenstoffatomen, Aralkanolen mit 7 oder 8 Kohlenstoffatomen oder Phenolen mit 6 bis 8 Kohlenstoffatomen ab. Besonders bevorzugte Ausgangsstoffe sind 6-Hydroxycapronsäure $C_1$-$C_4$-Alkylester, insbesondere 6-Hydroxycapronsäuremethylester. Beispielhaft seien genannt 6-Hydroxycapronsäuremethylester, 6-Hydroxycapronsäureethylester, 6-Hydroxycapronsäure-n-propylester, 6-Hydroxycapronsäure-i-propylester, 6-Hydroxycapronsäure-n-butylester, 6-Hydroxycapronsäure-2-ethylhexylester, 6-Hydroxycapronsäurecyclohexylester oder 6-Hydroxycapronsäurephenylester.

Ein wesentliches Merkmal der Erfindung ist es, daß man 6-Hydroxycapronsäureester dampfförmig zusammen mit einem Trägergas über fest angeordnete oder in auf- und abwirbelnder Bewegung befindliches Siliciumdioxid, z.B. in Form von Kiegelgel, Kieselgur oder Quarz, leitet.

6-Hydroxycapronsäureester werden vorteilhaft bei einer Temperatur von 180 bis 300°C verdampft. Hierbei hat es sich als vorteilhaft erwiesen, zusätzlich unter Reaktionsbedingungen inerte Lösungsmittel wie Ether, z.B. Dioxan oder Tetrahydrofuran, mitzuverdampfen. Vorteilhaft verwendet man 50 bis 90 gew.%ige Lösungen von 6-Hydroxycapronsäureester in solchen Lösungsmitteln.

Inerte Trägergase sind beispielsweise Stickstoff, Kohlendioxid oder Argon. Vorzugsweise wird Stickstoff als Trägergas verwendet. In der Regel verwendet man je Mol dampfförmigen 6-Hydroxycapronsäureester 10 bis 30 Mol Trägergas.

Nach einer Arbeitsweise wird Siliciumdioxid in der Reaktionszone fest angeordnet und das dampfförmige Gemisch aus 6-Hydroxycapronsäureestern und Trägergasen darübergeleitet. Nach einer anderen Arbeitsweise befindet sich der Katalysator in auf- und abwirbelnder Bewegung (Wirbelbett). Vorteilhaft hält man eine Katalysatorbelastung von 0,01 bis 40, insbesondere 0,1 bis 20 g Hydroxycapronsäureester je g Katalysator und Stunde ein.

Die Umsetzung wird bei einer Temperatur von 150 bis 450°C, vorzugsweise bei 200 bis 400°C, insbesondere 300 bis 350°C durchgeführt. Im allgemeinen wird die Reaktion unter Atmosphärendruck durchgeführt. Es ist jedoch auch möglich, schwach verminderten Druck, z.B. bis zu 500 mbar oder schwach erhöhten Druck, z.B. bis zu 1,2 bar anzuwenden.

Das Verfahren nach der Erfindung führt man beispielsweise durch, indem man 6-Hydroxycapronsäureester gegebenenfalls im Gemisch mit den genannten Lösungsmitteln verdampft und zusammen mit einem der genannten Inertgase bei den angegebenen Reaktionstemperaturen in eine in auf- und abwirbelnder Bewegung befindliche Katalysatorschicht der genannten Zusammensetzung einleitet. Der Reaktionsaustrag wird mit geeigneten Kühlvorrichtungen kondensiert und anschließend durch Destillation in bekannter Weise aufgearbeitet. Nicht umgesetzte 6-Hydroxycapronsäureester werden zweckmäßig wiederum für die Umsetzung

2

verwendet.

Das nach dem Verfahren der Erfindung erhältliche Caprolacton eignet sich zur Herstellung von Polyestern.

Das Verfahren nach der Erfindung sei an folgenden Beispielen veranschaulicht. Im folgenden bedeuten 6-HCE 6-Hydroxycapronsäuremethylester und CLO Caprolacton.

Beispiel 1

Pro Stunde wurden 10 ml 6-HCE in einen Verdampfer gepumpt und von dort gasförmig zusammen mit 3 l Stickstoff bei 250 bis 350°C über 10 g Siliciumdioxid (jeweils 1 bis 3 mm Splitt) geleitet. Die gasförmigen Reaktionsausträge wurden in Kühlfallen kondensiert, gewogen, und gaschromatographisch analysiert. In der Tabelle 1 ist die Ausbeute in Abhängigkeit von Temperatur und Katalysator nach jeweils 4 Stunden Versuchszeit angegeben.

Tabelle 1

| Nr | Temp. [°C] | Umsatz [mol.%] | Ausb. an CLO [mol.%] |
|----|------------|----------------|----------------------|
| 1 | 250 | 60 | 55 |
| 2 | 330 | 89 | 82 |
| 3 | 350 | 90 | 79 |

Beispiel 2

Pro Stunde wurden 10 ml 6-HCE bei ca. 300°C verdampft und mit 30 l Stickstoff über 45 g Siliciumdioxid-Wirbelkontakt (Korngröße 0,1 bis 0,3 mm) (Reaktionstemperatur 330°C) geleitet. Der gasförmige Reaktionsaustrag wurde in Kühlfallen kondensiert, gewogen und gaschromatographisch analysiert. Insgesamt wurden die Austräge über eine Versuchszeit von 72 Stunden gesammelt (Gesamt-Zulauf an 6-HCE: 757 g). Der flüssige Reaktionsaustrag (737 g) enthielt nach gaschromatographischer Analyse 66 Gew.% CLO, 10 Gew.% 6-HCE und 19 Gew.% Methanol.

Zur Aufarbeitung wurden die leichtersiedenden Anteile des Reaktionsaustrages (Methanol) im Vakuum abgezogen und der Rückstand im Vakuum fraktionierend destilliert.

Ausbeute: 437 g (80 % d.Th.) reines Caprolacton vom Sdp. 62°C/0,3 mbar.

Beispiel 3

Pro Stunde wurden 10 ml 6-HCE bei ca. 300°C verdampft und mit 30 l Stickstoff über 45 g Siliciumdioxid-Wirbelkontakt (Korngröße 0,1 bis 0,3 mm) (Reaktionstemperatur 330°C) geleitet.

Der gasförmige Reaktionsaustrag wurde in Kühlfallen kondensiert, gewogen und gaschromatographisch analysiert. Insgesamt wurden die Austräge über eine Versuchszeit von 36 Stunden gesammelt (Gesamtzulauf: 380 g 6-HCE). Der flüssige Reaktionsaustrag (362 g) enthielt nach gaschromatographischer Analyse 66 Gew.% CLO, 10 Gew.% 6-HCE und 19 Gew.% Methanol.

Zur weiteren Verarbeitung wurden die leichtersiedenden Anteile des Reaktionsaustrages (Methanol) im Vakuum abgezogen und der Rückstand (310 g) unter gleichen Bedingungen wie oben angegeben über den Siliciumdioxid-Wirbelkontakt geleitet.

Der gasförmige Reaktionsaustrag wurde erneut in Kühlfallen kondensiert, gewogen und gaschromatographisch analysiert. Der flüssige Reaktionsaustrag (295 g) enthielt jetzt nach gaschromatographischer Analyse 88 Gew.% CLO, 1 Gew.% 6-HCE und 2 Gew.% Methanol.

Zur Aufarbeitung wurden die leichtersiedenden Anteile des Reaktionsaustrages (Methanol) im Vakuum abgezogen und der Rückstand im Vakuum fraktionierend destilliert.

Ausbeute: 255 g (86 % d.Th.) reines Caprolacton vom Sdp. 62°C/0,3 mbar.

Beispiel 4

Pro Stunde wurden 10 ml 6-HCE bei ca. 300°C verdampft und mit 25 l Stickstoff über 45 g Siliciumdioxid-Wirbelkontakt (Korngröße 0,1 bis 0,3 mm) (Reaktionstemperatur 330°C) geleitet.

Der gasförmige Reaktionsaustrag wurde über eine Versuchsdauer von 168 Stunden in Kühlfallen kondensiert, gewogen und gaschromatographisch analysiert. Der flüssige Reaktionsaustrag enthielt nach gaschromatographischer Analyse durchgängig 71 Gew.% CLO, 5 Gew.% 6-HCE und 20 Gew.% Methanol (entsprechend einer CLO-Ausbeute von 89 % d.Th.).

## Patentansprüche

1. Verfahren zur Herstellung von $\varepsilon$-Caprolacton, bei dem man 6-Hydroxycapronsäureester in Gegenwart von oxidischen Katalysatoren auf eine Temperatur von 150 bis 450°C erhitzt, dadurch gekennzeichnet, daß man dampfförmige 6-Hydroxycapronsäureester zusammen mit einem inerten Trägergas über fest angeordnetes oder in auf- und abwirbelnder Bewegung befindliches Siliciumdioxid als Katalysator leitet.

2. Verfahren nach Anspruch 1, dadurch gekennzeichnet, daß man eine Temperatur von 300 bis 350°C einhält.

3. Verfahren nach den Ansprüchen 1 und 2, dadurch gekennzeichnet, daß man eine Katalysatorbelastung von 0,1 bis 20 g 6-Hydroxycapronsäureester je g Katalysator und Stunde einhält.

4. Verfahren nach den Ansprüchen 1 bis 3, dadurch gekennzeichnet, daß man je Mol 6-Hydroxycapronsäureester 10 bis 30 Mol Trägergas verwendet.

5. Verfahren nach den Ansprüchen 1 bis 4, dadurch gekennzeichnet, daß man 6-Hydroxycapronsäure-$C_1$-$C_4$-alkylester als Ausgangsstoffe verwendet.

## Claims

1. A process for the preparation of $\varepsilon$-caprolactone, in which a 6-hydroxycaproic ester in the presence of an oxidic catalyst is heated at from 150 to 450°C, which comprises passing the 6-hydroxycaproic ester in vapor form together with an inert carrier gas over a fixed-bed or fluidized-bed silica catalyst.

2. A process as claimed in claim 1, wherein a temperature of from 300 to 350°C is maintained.

3. A process as claimed in claims 1 and 2 wherein a space velocity of from 0.1 to 20 g of 6-hydroxycaproic ester per g of catalyst per hour is maintained.

4. A process as claimed in claims 1 to 3, wherein from 10 to 30 moles of carrier gas are used per mole of 6-hydroxycaproic ester.

5. A process as claimed in claims 1 to 4, wherein a $C_1$-$C_4$-alkyl 6-hydroxycaproate is used as the starting material.

## Revendications

1.- Procédé de préparation d'$\varepsilon$-caprolactone, dans lequel on chauffe des esters d'acide 6-hydroxycaproïque à une température de 150 à 450°C en présence de catalyseurs du type oxyde, caractérisé en ce qu'on fait passer l'ester d'acide 6-hydroxycaproïque à l'état de vapeur, en même temps qu'un gaz vecteur inerte, sur du dioxyde de silicium servant de catalyseur, disposé en lit fixe ou mis en mouvement tourbillonnaire vers le haut et vers le bas.

2.- Procédé selon la revendication 1, caractérisé en ce qu'on maintient une température de 300 à 350°C.

3. - Procédé selon la revendication 1 ou 2, caractérisé en ce qu'on maintient une charge du catalyseur de 0,1 à 20 g d'ester d'acide 6-hydroxycaproïque par gramme de catalyseur et par heure.

4.- Procédé selon l'une quelconque des revendications 1 à 3, caractérisé en ce qu'on utilise 10 à 30 moles de gaz vecteur par mole d'ester d'acide 6-hydroxycaproïque.

5.- Procédé selon l'une quelconque des revendications 1 à 4, caractérisé en ce qu'on utilise, comme substances de départ, les esters alkyliques en $C_1$-$C_4$ d'acide 6-hydroxycaproïque.